Europäisches Patentamt

European Patent Office

Office européen des brevets

(19)

(11) Publication number: **0 334 076 B1**

(12) **EUROPEAN PATENT SPECIFICATION**

(45) Date of publication of patent specification: **12.01.94**

(51) Int. Cl.5: **C12P 21/00**, C12N 5/00, G01N 33/68, G01N 33/577

(21) Application number: **89103754.1**

(22) Date of filing: **03.03.89**

(54) **Monoclonal antibody capable of recognizing an antigen associated with human arteriosclerosis, and process for preparing the same.**

(30) Priority: **03.03.88 JP 50162/88**

(43) Date of publication of application:
**27.09.89 Bulletin 89/39**

(45) Publication of the grant of the patent:
**12.01.94 Bulletin 94/02**

(84) Designated Contracting States:
**AT BE CH DE ES FR GB GR IT LI LU NL SE**

(56) References cited:
**EP-A- 0 236 637**
**WO-A-86/04090**
**US-A- 4 343 734**

**ARCHIVES OF PATHOLOGY AND LABORA-TORY MEDICINE, vol. 109, May 1985, pages 445-449, Chicago, US; D.M. KLURFELD: "Identification of foam cells in humanatherosclerotic lesions as macrophages using monoclonal antibodies"**

(73) Proprietor: **Takano, Tatsuya**
**432-44-3, Teradamachi**
**Hachioji-shi Tokyo(JP)**

(73) Proprietor: **DAIICHI RADIOISOTOPE LABORA-TORIES, LTD.**
**17-10, Kyobashi 1-chome**
**Chuo-ku Tokyo(JP)**

Proprietor: **NIHON MEDI-PHYSICS CO., LTD.**
**No 4-2-1, Takatsukasa**
**Takarazuka-shi**
**Hyogo-ken(JP)**

(72) Inventor: **Takano, Tatsuya**
**432-44-3, Teradamachi**
**Hachioji-shi Tokyo(JP)**
Inventor: **Takatoku, Keizo**
**1-24-2, Hiyoshidai**
**Tomisatomachi**
**Inba-gun Chiba-ken(JP)**

(74) Representative: **Hansen, Bernd, Dr.rer.nat. et al**
**Hoffmann, Eitle & Partner**
**Patentanwälte**
**Postfach 81 04 20**
**D-81904 München (DE)**

EP 0 334 076 B1

## Description

The present invention relates to a human arteriosclerosis recognizing monoclonal antibody that specifically recognizes a human arteriosclerosis related antigen. The present invention also relates to a process for preparing such monoclonal antibody. More particularly, the present invention provides a monoclonal antibody capable of recognizing a human arteriosclerosis related antigen that is useful in such applications as the determination of a human arteriosclerosis related antigen and diagnosis of arteriosclerosis based thereon, as well as imaging diagnosis of human arteriosclerotic lesions.

Arteriosclerosis is a localized disease developed principally in large or middle sized arterias such as the aorta, coronary artery and cerebral artery and is a major cause of various occlusive diseases such as angina pectoris, myocardial infarction and cerebral infarction.

Many phenomena have so far been proposed as causes of arteriosclerosis and they include increased plasma cholesterol levels, damage to the endothelium, aggregation of blood platelets, hyperplasia of the tunica intima and the formation of atheroma. However, little analysis has been made of the exact mechanism of these phenomena.

The normal aorta has a three-layered structure composed of an endothelium, a tunica media made of elastic tissues and smooth muscle cells, and a tunica externa made of elastic tissues. If, for some reason, the boundary portion between the endothelium and the tunica media expands and abnormal cell proliferation and necrosis occur, a disease will develop which is generally referred to as arteriosclerosis.

Among the principal causes of serious diseases such as myocardial infarction and cerebral infarction are:

(1) formation of atheroma due to the accumulation of cholesterol and other lipids in and between arterial wall cells;

(2) hyperplasia of the tunica intima as a result of abnormal cell proliferation; and

(3) aggregation of blood platelets as a result of damage to the endothelium and hyperplasia of the tunica intima.

Because of these reasons, arterial vessels are occluded, presumably causing the development of such diseases as angina pectoris, myocardial infarction and cerebral infarction.

The following phenomena are typically found to have occurred in the expanded portion of the tunica intima:

(a) the appearance of foam cells taking up a large amount of lipids therein;

(b) accumulation of lipids between cells;

(c) proliferation of smooth muscle cells in the tunica intima;

(d) increased formation of connective tissues and the deposition of calcium; and

(e) aggregation of blood platelets and formation of thrombi.

Diagnosis of human arteriosclerosis can be made either directly or indirectly. Indirect methods involve predicting the degree of risk based on the measurement of cholesterol levels in blood, analysis of lipoprotein composition and search for coagulation factors. Among the direct methods are echography techniques in which the progress of arteriosclerosis is estimated by measuring the velocity of sound propagating through the arterial wall or by utilizing echoes reflected by ultrasonic vibrations from the arterial wall, and angiography techniques in which an angiogram of the artery obtained by injecting an imaging medium into the arterial vessel is directly examined.

The determination of risk factors in blood as effected by the indirect methods does not involve the measurement of direct causative factors of arteriosclerosis and hence is not considered to be a highly reliable method of diagnosis. The direct methods, whether they are echographic or angiographic, involve the measurement of the degree of blood vessel's constriction due to arteriosclerosis and are not directed to measuring the progress of the disease itself. Furthermore, the angiographic technique has a potential hazard on account of the need to inject an imaging medium into arteries.

EP-A-0 236 637 discloses a monoclonal antibody capable of specifically recognizing arteriosclerotic lesions and obtained from a hybridoma which is produced by fusing myeloma cells and cells capable of producing antibody against arteriosclerotic lesions.

It is desired to develop an index material which is convenient and has high specificity for human arteriosclerosis.

The present inventors conducted extensive studies on factors that would act directly on human arteriosclerosis. As a result, the present inventors, using sera from arteriosclerotic patients or sites of arteriosclerotic lesions as antigens, isolated monoclonal antibody producing cells that would specifically recognize antigens related to human arteriosclerotic diseases such as familial hypercholesterolemia, myocardial infarction and cerebral infarction, and successfully obtained from these cells a monoclonal antibody capable of recognizing human arteriosclerosis. Therefore in one aspect, the present invention relates to a monoclonal antibody designated as 125H or 131B as obtainable by hybridoma cell lines FERM BP 1675 and 1676. Said monoclonal antibody is capable of specifically recognizing a human arteriosclerosis related antigen.

Furthermore, the present invention relates to a process for preparing a human arteriosclerosis recognizing monoclonal antibody as claimed in any of claims 4 to 8.

In an advantageous convenient method, the monoclonal antibody of the present invention can be produced from hybridomas prepared by fusing anti-human arteriosclerosis antibody producing cells with myeloma cells. This method of production may start with sensitization of a non-human mammal by standard techniques using a human arteriosclerosis antigen. Illustrative human arteriosclerosis antigens include sera isolated from patients with human arteriosclerotic diseases such as familial hypercholesterolemia, cerebral infarction and myocardial infarction, and a homogenate of arteriosclerotic lesion sites (in particular, a swelling portion of the tunica intima). When the sera are used as the antigens, they may be preferably mixed with anti-normal plasma anti-sera as illustrated in Example 1 hereunder whereby the subsequent sensitization can be efficiently conducted. In the next step of the method, lymphocytes capable of producing an anti-human arteriosclerosis antibody are isolated from the spleen of the sensitized animal, especially, the thymus, peripheral lymph nodes or peripheral blood, thereby obtaining cells capable of producing an anti-human arteriosclerosis antibody. These cells are then fused with myeloma cells by standard techniques to obtain antibody producing hybridomas. The hybridomas are distributed among a plurality of wells and cultivated. The supernatant of the culture in each well is assayed by a suitable technique such as enzyme immunoassay (ELISA) or indirect fluorescent antibody technique, so as to isolate human arteriosclerosis recognizing monoclonal antibody producing cells that bind specifically to the serum of a human arteriosclerotic patient or a human arteriosclerotic lesion and which does not recognize the normal human serum or normal arterial wall. The isolated cells are directly subjected to tissue cultivation. Alternatively, they may be transplanted in the abnormal cavity of a mammal, say, a mouse or guinea pig, to produce a tumor. The desired monoclonal antibody is collected from the ascites and purified.

The present inventors thus succeeded in isolating novel human arteriosclerosis recognizing monoclonal antibodies that specifically recognize human arteriosclerosis related antigens.

Among the monoclonal antibodies obtained in the present invention, monoclonal antibody 131B that was produced using sera from patients with hypercholesterolemia, cerebral infarction and myocardial infarction as antigens is capable of specifically recognizing the arteriosclerosis related antigens found in sera from human patients and is classified as IgG.

Monoclonal antibody 125H could be obtained using as antigens the homogenates of hyperplasia portions of the tunica intima of arteriosclerotic patients. Monoclonal antibody 125H which is classified as IgG was found to be capable of specifically recognizing arteriosclerotic lesions.

Fig. 1 is a photograph showing the results of immunoglobulin classification of monoclonal antibody 131B by the Ouchterlony method;

Fig. 2 is a chart plotting the antigenic activities by disease of monoclonal antibody 131B based on the data shown in Table 1;

Fig. 3 is a chart showing the results of protein and immunofluorescent staining of arteriosclerosis related substances recognized by monoclonal antibody 131B;

Fig. 4 is a graph showing antigenic substances recognized by monoclonal antibody 131B;

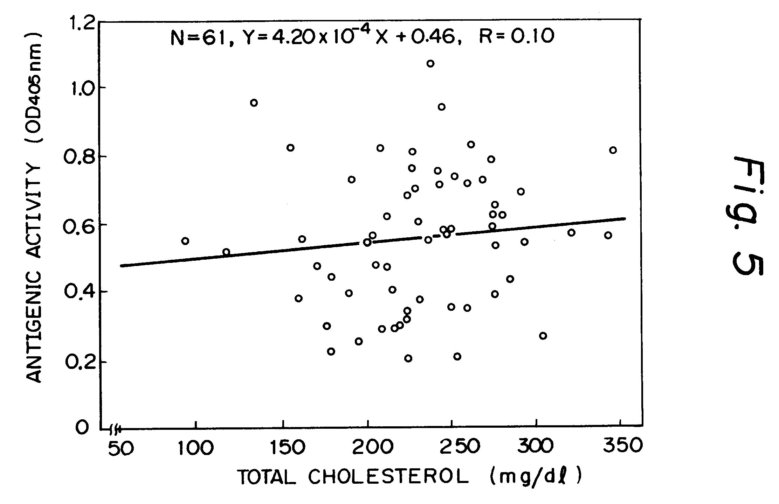

Fig. 5 is a graph showing the correlationship between antigenic substances recognized by monoclonal antibody 131B and cholesterol levels in familial hypercholesterolemic patients;

Fig. 6 is a chart plotting the antigenic activities by disease of monoclonal antibody 125H based on the data shown in Table 2;

Fig. 7 is a micrograph showing the results of immunofluorescent staining by the indirect antibody technique of a frozen section of an arteriosclerotic lesion in rabbit that was allowed to react with monoclonal antibody 125H;

Fig. 8 is a micrograph showing the results of staining with Oil-Red O of a frozen section of an arteriosclerotic lesion in rabbit;

Fig. 9 is a photograph showing the state of reaction between an arteriosclerotic lesion in rabbit and monoclonal antibody 125H as observed by indirect autoradiography;

Fig. 10 is a micrograph showing the state of the normal portion that was allowed to react with monoclonal antibody 125H and which was observed by indirect autoradiography; and

Fig. 11 is a photograph showing the results of protein and immunofluorescent staining of antigenic substances recognized by monoclonal antibody 125H.

The monoclonal antibodies of this invention 131B and 125H were obtained by sensitization using sera from human patients or homogenates of the arterial wall as antigens and all of them were novel in that they differed from the monoclonal antibodies prepared by using rabbit sera and homogenates of the arterial wall as antigens.

The monoclonal antibodies of the present invention may be immediately used as reagents for estimating the progress of human arteriosclerosis. If desired, these antibodies may be treated with proteolytic enzymes and the resulting decomposition products, F(ab')$_2$ and Fab, used as reagents for estimating the progress of human arteriosclerosis. It is interesting to note that the antigens recognized by these monoclonal antibodies occur not only in human arteriosclerotic lesion sites but also in sera from arteriosclerotic patients. Therefore, quantitative or qualitative analyses of the antigens present in sampled sera can be performed, using these monoclonal antibodies as reaction reagents, by a variety of known techniques including immunoassay techniques (e.g. competitive and sandwich methods) using markers such as enzymes (e.g. alkaline phosphatase, $\beta$-galactosidase and peroxidase), radioisotopes and fluorescent materials, by measurements depending on agglutination reaction or reaction for inhibiting agglutination, or by modifications of these methods. The results of these analyses can be used to estimate the progress of arteriosclerosis of interest.

The monoclonal antibodies of the present invention can also be used to detect the presence of a human arteriosclerotic lesion site or to check the extent of its spread. For instance, using radioisotopes such as Na$^{131}$I, Na$^{123}$I and Na$^{125}$I, iodine is bound to these monoclonal antibodies or fragments thereof such as F-(ab')$_2$ and Fab by such methods as the chloramine-T method and enzyme method. Technetium may be bound by adding a physiological saline solution of Na$^{99m}$TcO$_4$ (sodium pertechnetate) in the presence of a suitable reducing agent such as SnCl$_2$. Alternatively, indium ($^{111}$In) may be bound with the aid of a suitable chelating agent [so-called bifunctional chelate such as diethylenetriamine pentaacetic acid (DTPA) anhydride]. The monoclonal antibodies or fragments thereof to which iodine, technetium or indium has been bound are then added to a sterile nontoxic medium and injected into veins. After a certain period of time, the results of association of the monoclonal antibodies or fragments thereof with the arteriosclerotic lesion are examined with an imaging device such as a gamma-camera to obtain a scintigram based on the distribution of radioactivity in the patient's body. The scintigram can be used to detect the presence of lesions of human arteriosclerotic diseases such as cerebral infarction and myocardial infarction or to check the extent of their spread.

The following examples are provided for the purpose of further illustrating the present invention.

Example 1  Monoclonal Antibody of Class IgG Prepared Using Sera from Hyperlipidemic Patients as Antigens

(1) Preparation of monoclonal antibodies

A portion (20 $\mu$l) of blood plasma taken from a normal subject was diluted 10 folds with 180 $\mu$l of physiological saline and administered intraperitoneally into BALB/c mice. Following this injection, the same dilution was administered intraperitoneally after 3 weeks and 2 months, thereby obtaining anti-normal plasma anti-sera. Sera taken from 19 familial hyperlipidemic patients were mixed together and 20 $\mu$l of the mixture were diluted 5 fold with 80 $\mu$l of physiological saline. The dilution was intimately mixed with 100 $\mu$l of a Freund's complete adjuvant and the resulting emulsion was administered subcutaneously to BALB/c mice. After 2 weeks, 10 $\mu$l of the previously prepared anti-normal plasma anti-sera and 10 $\mu$l of a mixed serum from 19 familial hyperlipidemic patients were diluted with 80 $\mu$l of physiological saline. The dilution was intimately mixed with 100 $\mu$l of a Freund's complete adjuvant and the resulting emulsion was administered subcutaneously into the animals. After 2 weeks, the mice were finally immunized by intraperitoneal injection of a dilution in physiological saline (180 $\mu$l) of 10 $\mu$l of the anti-normal plasma anti-sera and 10 $\mu$l of a mixed serum of 19 familial hyperlipidemic patients. On the third day, spleens were extracted from the sensitized animals.

The spleen cells were thoroughly washed with HHBS (Hepes buffered Hanks' balanced salt solution), mixed with thoroughly washed mouse myeloma cells (strain P3/UI) at a ratio of 5:1, and centrifuged at 1,300 rpm for 5 minutes. The cell pellet was suspended in 1 ml of DMEM(-) medium containing 50% polyethylene glycol 4000 and left to stand for 2 minutes. Subsequently, 10 ml of DMEM(-) medium was slowly added to make a dilution, which was centrifuged at 800 rpm for 5 minutes. The cell pellet was resuspended in 100 ml of a HAT medium containing 20% fetal calf serum and the suspension was distributed in 0.1-ml portions among 96 wells in a tissue culture plate. Every 2 - 4 days, half the medium was replaced with a fresh medium. Investigation of antibody titer conducted on the supernatants of the culture after 8 days showed

4

that strong antibody activities were found in 21 of 1,000 wells. Cloning was then performed by a limiting dilution technique so as to select clones that would not show any antibody activity against a mixed plasma of 14 normal subjects or a mixed serum of 14 normal subjects but which would specifically recognize arteriosclerosis related antigens in human sera. As a result, a total of 9 clones of hybridomas were obtained. The final cloning was conducted by a limiting dilution technique so as to isolate a total of 6 clones of hybridomas that were more inactive against normal plasma and sera but which specifically reacted with sera from patients suffering from arteriosclerosis related diseases. These clones were injected intraperitoneally into pristan-treated BALB/c mice and after 10 - 20 days, ascites was collected from each animal to obtain monoclonal antibodies.

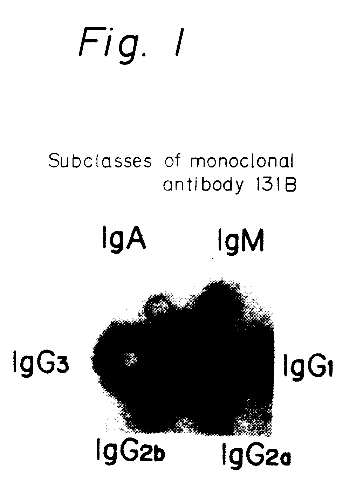

Of the monoclonal antibodies obtained from the 6 clones of hybridomas, those obtained from cell line 131B were found to be of immunoglobulin subclass $IgG_1$ by the Ouchterlony method (see Fig. 1) and they had the ability to specifically recognize arteriosclerosis related antigens in human sera. Cell line 131B has been deposited under the Budapest Treaty with the Fermentation Research Institute (FERM) the Agency of Industrial Science and Technology, an international depository authority, under Accession Number FERM BP-1676.

(2) Assaying patient sera with monoclonal antibody 131B

The sera to be tested were as follows: serum from a familial hyperlipidemic patient (FH); serum from type III hyperlipidemic patient (Type III); serum from hypo-betalipoproteinemic patient (Hypo $\beta$); serum from patient with myocardial infarction (MI); serum from patient with cerebral infarction (APO); serum from patient with Werner's syndrome (Werner); serum from patient with angina pectoris (Angina); plasma from normal subject (NP); and a mixed serum prepared from blood samples of 14 normal subjects (NS). Each of these sera was diluted 1,000 fold and 100 $\mu$l of each dilution was added to an ELISA microplate (Falcon 3912). The plate was left to stand overnight at 4°C to have the homogenate adsorbed on it. After treating the microplate with a phosphate buffer solution containing 1% BSA, 100 $\mu$l of a solution of monoclonal antibody 131B was added and reaction was performed at 37°C for 2 hours. After washing the microplate, 100 $\mu$l of 10,000-fold diluted alkaline phosphatase labelled anti-mouse IgG (Tago) was added and reaction was carried out at 37°C for 1 hour. After washing the microplate, 100 ml of 1 mM diethanolamine buffer solution (pH, 9.8) containing 1 mg/ml of disodium salt of paranitrophenylphosphoric acid and 0.01% $MgCl_2$ were added and reaction was carried out at 37°C for 2 hours. Optical absorbance values ($OD_{405}$ nm) measured with a microplate colorimeter (Bio-Rad) were used as indices of activity against arteriosclerosis related antigens. The results are shown in Table 1. The monoclonal antibody 131B of the present invention exhibited high activity levels through highly specific recognition of arteriosclerosis related antigens in FH, Type III, Hypo $\beta$, MI, APO, Werner and Angina. In addition, this monoclonal antibody showed only low activity levels against NP and NS. It was, therefore, clear that monoclonal antibody 131B was capable of specifically recognizing arteriosclerosis related antigens in human sera.

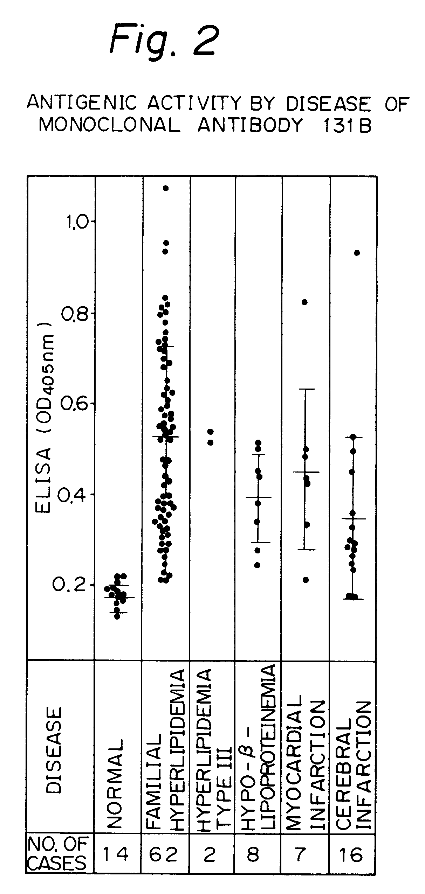

These results are shown graphically in Fig. 2, from which one can see that all sera from hypercholesterolemic patient, Type III hyperlipidemic patient, hypo-betalipoproteinemic patient, myocardial infarction patient and cerebral infarction patient showed higher antigenic activity levels than normal plasma and sera.

(3) Arteriosclerosis related antigenic substances in human serum capable of reacting with monoclonal antibody 131B

Sera from familial hypercholesterolemic patients were subjected to electrophoresis through an SDS-added polyacrylamide gel overnight at 40 volts and blotted on a nitrocellulose membrane. The membrane was thoroughly washed with a phosphate buffer solution containing 0.05% nonionic surfactant Tween 20 and thereafter treated overnight with a phosphate buffer solution containing 5% BSA. After washing, the membrane was subjected to reaction with monoclonal antibody 131B for 2 hours, then with alkaline phosphatase labelled anti-mouse IgG antibody for 1 hour. After the reaction, the membrane was immersed in a 0.75 M Tris-HCl buffer solution (pH, 8.8) containing 0.2% paratoluidine salt of 5-bromo-4-chloro-3-indolylphosphoric acid and left to stand overnight at room temperature for immunofluorescent staining. It was estimated from the results of staining that the arteriosclerosis related antigenic substances in human serum that were specifically recognized by monoclonal antibody 131B had molecular weights of approximately 52,000.

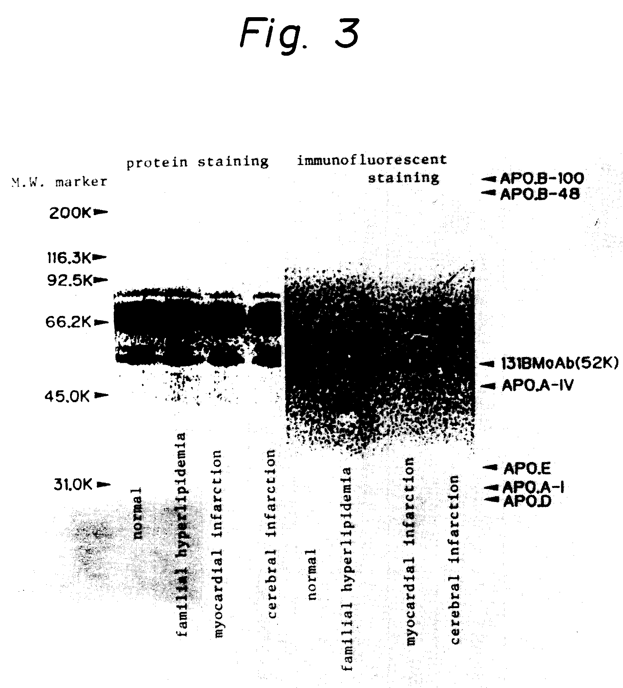

The antigenic substances recognized by monoclonal antibody 131B have specificity for hyperlipidemia and differ from lipoprotein constituents, such as Apo A-I, A-IV, B-100, B-48, E and D, which are said to be closely related to arteriosclerosis (see Fig. 3).

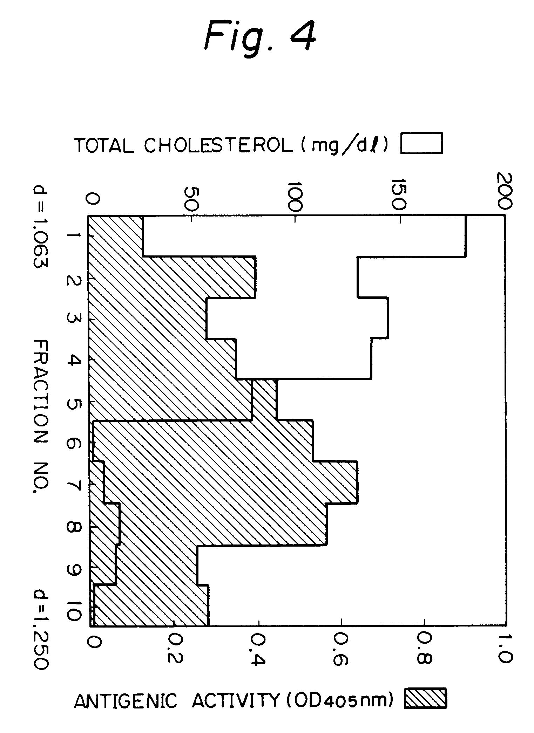

As shown in Fig. 4, the antigenic substances recognized by monoclonal antibody 131B were found in fractions that did not float even after 24-hour centrifugation at 30,000 rpm of 1 ml of serum from a familial hyperlipidemic patient that had been adjusted to a specific gravity of 1.25 with NaBr and thereafter plated with 2 ml of physiological saline having a specific gravity of 1.153 and 1.6 ml of physiological saline having a specific gravity of 1.063. Furthermore, the low degree of correlation (R = 0.104) with the cholesterol level of familial hyperlipidemic patients showed that monoclonal antibody 131B does not recognize LDL or lipoproteins as antigenic substances.

From these results, it is concluded that monoclonal antibody 131B specifically recognizes those antigenic substances which are characteristic of arteriosclerotic diseases and which are commonly found in patient's sera. It has also been found that this monoclonal antibody is capable of specific recognition of antigenic substances in blood plasma, too.

Table 1

Cholesterol Levels of Sera from Patients Suffering
from Arteriosclerosis Related Diseases and
Their Antigenic Activities for Monoclonal Antibody 131B

| Disease | Sample No. | Patient No. | T.C. (mg/dl) | ELISA (OD$_{405}$ nm) |
|---------|-----------|-------------|--------------|-----------------------|
| FH | 1 | 73-02615 | 91 | 0.551 |
| FH | 2 | 81-06627 | 159 | 0.372 |
| FH | 3 | 80-48341 | 219 | 0.300 |
| FH | 4 | 85-12795 | 176 | 0.299 |
| FH | 5 | 78-01909 | 179 | 0.223 |
| FH | 6 | 81-42373 | 223 | 0.340 |
| FH | 7 | 82-59757 | 188 | 0.399 |
| FH | 8 | 82-60373 | 232 | 0.376 |
| FH | 9 | 83-11984 | 223 | 0.314 |
| FH | 10 | 85-18298 | 216 | 0.292 |
| FH | 11 | 81-42373 | 195 | 0.259 |
| FH | 12 | 82-57486 | 225 | 0.205 |
| FH | 13 | 78-03839 | 320 | 0.570 |
| FH | 14 | 78-00694 | 285 | 0.434 |
| FH | 15 | 86-26248 | 245 | 0.580 |
| FH | 16 | 81-58703 | 244 | 0.937 |
| FH | 17 | 86-13848 | 276 | 0.650 |
| FH | 18 | 85-46484 | 250 | 0.351 |
| FH | 19 | 81-14720 | 274 | 0.629 |
| FH | 20 | 78-03537 | 274 | 0.591 |
| FH | 21 | 78-04523 | 241 | 0.747 |
| FH | 22 | 79-66446 | 258 | 0.715 |
| FH | 23 | 79-07111 | 260 | 0.832 |

Table 1 (cont'd)

| Disease | Sample No. | Patient No. | T.C. (mg/dl) | ELISA (OD$_{405}$ nm) |
|---|---|---|---|---|
| FH | 24 | 80-19562 | 241 | 0.712 |
| FH | 25 | 78-07831 | 345 | 0.808 |
| FH | 26 | 83-35843 | 342 | 0.561 |
| FH | 27 | 70-01093 | 236 | 1.069 |
| FH | 28 | 87-30840 | 304 | 0.269 |
| FH | 29 | 80-05283 | 223 | 0.681 |
| FH | 30 | 86-18468 | 229 | 0.603 |
| FH | 31 | 86-26301 | 212 | 0.471 |
| FH | 32 | 85-37071 | 178 | 0.446 |
| FH | 33 | 82-29714 | 215 | 0.401 |
| FH | 34 | 80-41131 | 276 | 0.535 |
| FH | 35 | 80-06511 | 254 | 0.211 |
| FH | 36 | 82-45798 | 211 | 0.621 |
| FH | 37 | 78-03381 | 260 | 0.349 |
| FH | 38 | 87-19069 | 292 | 0.546 |
| FH | 39 | 86-33547 | 205 | 0.478 |
| FH | 40 | 85-19289 | 247 | 0.573 |
| FH | 41 | 87-23854 | 268 | 0.724 |
| FH | 42 | 85-04596 | 227 | 0.697 |
| FH | 43 | 86-19522 | 273 | 0.785 |
| FH | 44 | 79-64322 | 170 | 0.475 |
| FH | 45 | 84-29516 | 226 | 0.807 |
| FH | 46 | 85-18616 | 153 | 0.822 |
| FH | 47 | 81-37201 | 235 | 0.549 |
| FH | 48 | 79-36564 | 202 | 0.565 |

Table 1 (cont'd)

| Disease | Sample No. | Patient No. | T.C. (mg/dl) | ELISA (OD$_{405}$ nm) |
|---|---|---|---|---|
| FH | 49 | 78-06522 | 225 | 0.756 |
| FH | 50 | 78-08511 | 209 | 0.291 |
| FH | 51 | 86-07503 | 199 | 0.546 |
| FH | 52 | 78-05045 | 278 | 0.622 |
| FH | 53 | 86-39926 | 250 | 0.586 |
| FH | 54 | 85-32090 | 162 | 0.558 |
| FH | 55 | 85-33074 | 1I5 | 0.521 |
| FH | 56 | 81-16704 | 190 | 0.727 |
| FH | 57 | 80-27988 | 251 | 0.737 |
| FH | 58 | 87-14349 | 290 | 0.689 |
| FH | 59 | 80-27368 | 207 | 0.818 |
| FH | 60 | 86-01605 | 276 | 0.390 |
| FH | 61 | 84-45499 | 131 | 0.951 |
| APO | 62 | 78-01164 | 106 | 0.247 |
| APO | 63 | 83-18926 | 123 | 0.534 |
| APO | 64 | 78-00900 | 115 | 0.939 |
| APO | 65 | 73-00300 | 163 | 0.259 |
| APO | 66 | 78-09966 | 148 | 0.186 |
| APO | 67 | 78-03785 | 163 | 0.360 |
| APO | 68 | 86-25083 | 187 | 0.282 |
| APO | 69 | 78-00849 | 199 | 0.271 |
| APO | 70 | 78-05165 | 185 | 0.296 |
| APO | 71 | 84-10579 | 205 | 0.496 |
| APO | 72 | 73-06841 | 117 | 0.451 |
| APO | 73 | 78-01373 | 131 | 0.302 |
| APO | 74 | 77-02200 | 170 | 0.291 |

Table 1 (cont'd)

| Disease | Sample No. | Patient No. | T.C. (mg/dl) | ELISA (OD$_{405}$ nm) |
|---|---|---|---|---|
| MI | 75 | 84-00571 | 164 | 0.335 |
| MI | 76 | 78-00485 | 161 | 0.499 |
| MI | 77 | 78-04516 | 133 | 0.433 |
| MI | 78 | 78-02674 | 177 | 0.423 |
| MI | 79 | 74-03633 | 178 | 0.483 |
| MI | 80 | 73-03652 | 105 | 0.823 |
| Hypo β | 81 | 82-21911 | 99 | 0.457 |
| Hypo β | 82 | 87-32118 | 91 | 0.506 |
| Hypo β | 83 | 78-03270 | 67 | 0.280 |
| Hypo β | 84 | 80-45830 | 93 | 0.346 |
| Hypo β | 85 | 84-26556 | 78 | 0.444 |
| Hypo β | 86 | 85-20771 | 114 | 0.372 |
| Hypo β | 87 | 83-13324 | 100 | 0.251 |
| Hypo β | 88 | 78-01437 | 101 | 0.517 |
| Type III | 89 | 79-31979 | 198 | 0.545 |
| Type III | 90 | 725900 | 243 | 0.520 |
| Werner | 100 | 85-40293 | 183 | 0.507 |
| Werner | 101 | 81-18040 | 147 | 0.447 |
| Angina | 102 | 83-20213 | 182 | 0.205 |

T.C.      : Total cholesterol
FH        : Familial hyperlipidemia
APO       : Cerebral infarction
MI        : Myocardial infarction
Hypo β    : Hypo-β-lipoproteinemia
Type III  : Hyperlipidemia type III
Werner    : Werner's syndrome
Angina    : Angina pectoris

Example 2 Arteriosclerotic Wall Recognizing Monoclonal Antibody of Class IgG Prepared Using Arteriosclerotic Lesions as Antigens

(1) Preparation of antigen

Human thoracic and abdominal arteriosclerotic walls were removed and blood vessels were excised in a cool place. Thereafter, the hyperplasia portion of the tunica intima (arteriosclerotic lesion) was peeled off with tweezers and cut into squire pieces (1 mm x 1 mm) with scissors. To these pieces, an aqueous solution (pH, 7.4) containing 1 mM EDTA and 0.1% ethanol was added in portions of 5 - 10 ml per gram on a wet weight basis and a homogenate was prepared with a polytron homogenizer.

A mixture of homogenates originating from 3 patients (equivalent to 5 specimens) was filtered through 4 superposed sheets of gauze and the filtrate was used as an antigenic solution.

(2) Preparation of monoclonal antibodies

To 0.5 mg (200 $\mu$l) of the homogenate, 200 $\mu$l of Freund's complete adjuvant was added and mixed well. The resulting emulsion was administered subcutaneously into BALB/c mice. After 9 weeks, the same emulsion was administered subcutaneously. After 18 weeks, the final immunization was conducted by intraperitoneal injection of a mixture of 0.5 mg (200 $\mu$l) of the homogenate. On the third day, spleens were extracted from the sensitized animals. The spleen cells were thoroughly washed with DMEM(-) medium and mixed with thoroughly washed mouse myeloma cells (strain P3/U1) at a ratio of 5:1 and the mixture was centrifuged at 1,000 rpm for 5 minutes.

The cell pellet was suspended in 1 ml of DMEM(-) medium containing 45% polyethylene glycol 4000 and left to stand for 2 minutes. Subsequently, 10 ml of DMEM(-) medium was slowly added to make a dilution, which was centrifuged at 1,000 rpm for 5 minutes. The cell pellet was resuspended in 127 ml of a DMEM medium containing 10% fetal calf serum and the suspension was distributed in 1-ml portions among 24 wells in a tissue culture plate.

On the first day of culture, 1 ml of a HAT medium was added and half the medium was replaced with a fresh medium every 1 - 2 days. On the 8th day, the antibody titers of the culture supernatants were examined by the ELISA method.

The previously prepared homogenate of the hyperplasia portion of the tunica intima of human arteriosclerotic lesion was diluted to make a solution having a protein concentration of 10 $\mu$g/ml. A 100-$\mu$l portion of this antigenic solution was added to an ELISA microplate (Nunc), which was left to stand overnight at 4°C to have the homogenate adsorbed on it. After treating the microplate with a 2% BSA solution, 100 $\mu$l of the culture supernatants from 127 wells were added and reaction was conducted for 2 hours at room temperature. After washing the microplate, 100 $\mu$l of 6,000-fold diluted alkaline phosphatase labelled anti-mouse IgG antibody (Tago) were added and reaction was carried out for 2 hours at room temperature. After washing the microplate, 100 $\mu$l of a 1 mM diethanolamine buffer solution (pH, 9.8) containing 1 mg/ml of disodium salt of paranitrophenylphosphoric acid and 0.01% $MgCl_2$ were added and reaction was carried out for 1 hour at room temperature. Optical absorbance ($OD_{405}$ nm) was measured with a microplate colorimeter (Bio-Tech). Sixty-five wells having absorbance values of 0.5 and higher were further examined by autoradiography.

Arteriosclerotic lesions were removed from arteriosclerotic model rabbits that had been grown with 1% cholesterol-supplemented diets for 4 months, and the lesions were cut into pieces. After treating these pieces with a 5% BSA solution, 1 ml of the culture supernatants from the 65 wells was added and reaction was performed at 4°C for 5 hours. After washing, the reaction mixture was treated with 5% normal sheep serum and 200 $\mu$l (1.7 Ci/ml) of $^{125}$I labelled anti-mouse IgG antibody (unlabelled; Tago) were added, followed by reaction at 4°C for 3 hours. After washing, the reaction mixture was subjected to autoradiography with X-ray films (Fuji Photo Film).

From the 65 wells tested, 10 wells that produced a specific reaction and exhibited high binding efficiencies were selected and subjected to cloning by a limiting dilution technique. As a result, a total of 2 clones of hybridoma was isolated. These hybridomas were injected intraperitoneally into pristan-treated BALB/c mice and after 10 - 16 days, ascites were collected from each animal to obtain monoclonal antibodies. One of these clones was 125H and was found to be immunoglobulin subclass $IgG_{2b}$ by the ELISA method and had the ability to specifically recognize arteriosclerotic plaque. Cell line 125H has been deposited under the Budapest Treaty with the FERM, an international depository authority, under Accession Number FERM BP-1675.

(3) Specificity of monoclonal antibody 125H

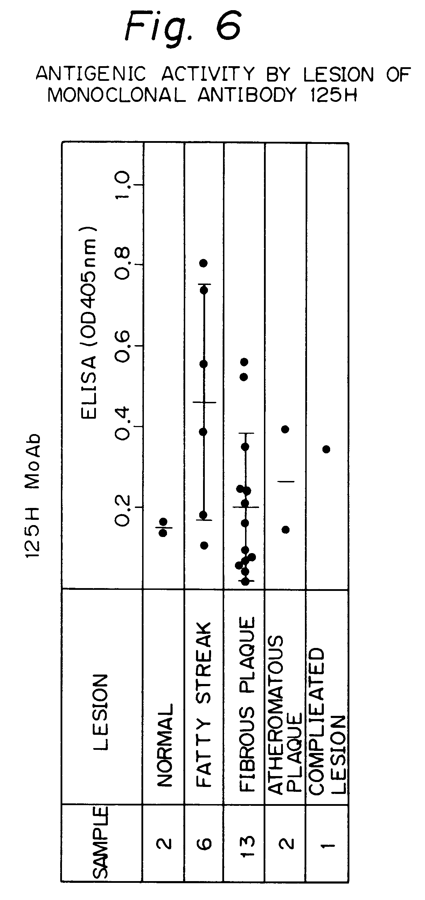

Using 100 $\mu$l of homogenates of the various human arteriosclerotic lesions of tunica intima and those of tunica intima of normal artery (each homogenate being adjusted to a protein content of 10 $\mu$g/ml), the reactivity of monoclonal antibody 125H was investigated by the ELISA method. The results are shown in Table 2 and Fig. 6. It was established that this monoclonal antibody shows strong activity with human arteriosclerotic lesions.

Measurements of total cholesterol (TC) levels were conducted with a cholesterol assay kit (V-cholestase "Nissui" of Nissui Seiyaku Co., Ltd.)

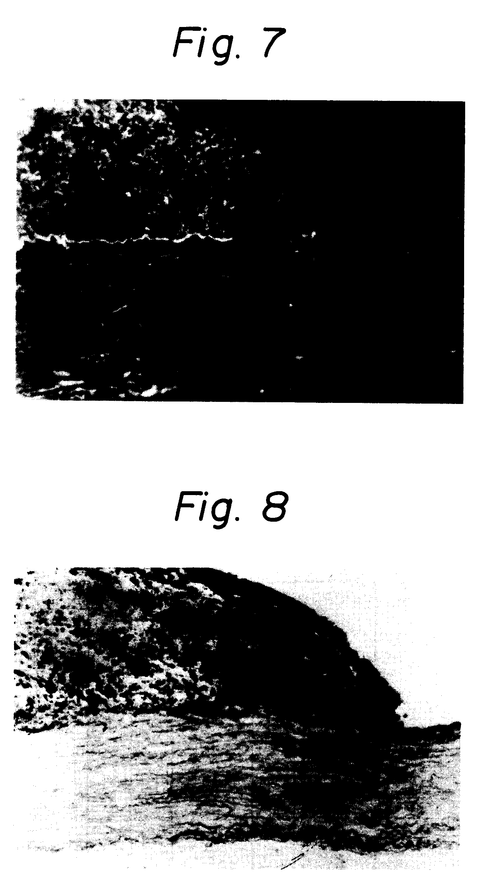

The reactivity of monoclonal antibody 125H with the hyperplasia portion of a rabbit arteriosclerotic tunica intima was also investigated. The results of immunofluorescent staining (by the indirect fluorescent antibody technique) conducted on frozen sections of the arteriosclerotic lesions in arteriosclerotic model rabbits that had been grown with 1% cholesterol-supplemented diets are shown in Fig. 7, and the results of staining with Oil-Red O conducted on frozen sections of the same lesions are shown in Fig. 8.

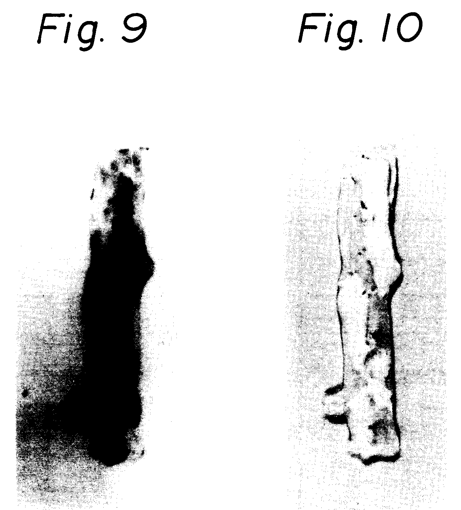

Arteriosclerotic lesions were also removed from another group of arteriosclerotic model rabbits that had been grown with 1% cholesterol-supplemented diets and the reactivity of monoclonal antibody with these lesions was examined by autoradiography. The normal portion was used as a control. The results of reaction by indirect autoradiography and the state of the lesions and the normal portion after the reaction are shown in Figs. 9 and 10, respectively. As is clear from these figures, monoclonal antibody 125H specifically recognized the hyperplasia portion of rabbit arteriosclerotic tunica intima.

On the basis of these results, one may be justified to expect that monoclonal antibody 125H will react with arteriosclerotic lesions after administration in vivo. When one wants to prepare a reagent for image analysis to be performed as an adjunct in the diagnosis of human arteriosclerosis, one is capable of conducting extensive preliminary tests on dosage, safety and other factors using rabbits before applying said reagent to humans. Therefore, monoclonal antibody 125H has a potential application in image analyses for the purpose of identifying the site of a specific human arteriosclerotic lesion and examining the extent of its spread.

(4) Antigenic substances in arteriosclerotic wall capable of reacting with monoclonal antibody 125H

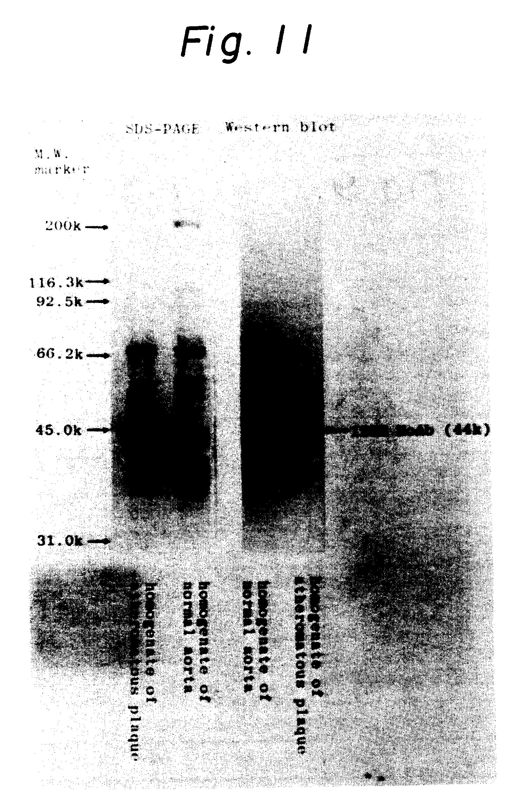

Homogenates of swelling tunica intima of arteriosclerotic lesions and those of a normal arterial wall were subjected to electrophoresis through an SDS-added polyacrylamide gel for 2 hours at 20 mA, and blotted on a nitrocellulose membrane. The membrane was thoroughly washed with a phosphate buffer solution containing 0.05% nonionic surfactant Tween 20 and thereafter treated with a 2% BSA solution for 6 hours. After washing, the membrane was subjected to reaction with monoclonal antibody 125H for 16 hours, then with alkaline phosphatase labelled anti-mouse IgG + IgM antibody for 2 hours. After the reaction, the membrane was immersed in a 0.75 M Tris-HCl buffer solution IgG + IgM antibody for 2 hours. After the reaction, the membrane was immersed in a 0.75 M Tris-HCl buffer solution (pH, 8.8) containing 0.1% paratoluidine salt of 5-bromo-4-chloro-3-indolylphosphoric acid and left to stand at room temperature for 2 hours. As a result, it was established that the antigenic substances recognized by monoclonal antibody 125H had molecular weights of approximately 44,000 (see Fig. 11).

Table 2
Specificity of Monoclonal Antibody 125H for
Arteriosclerotic Lesions

| | Subject No. | Disease | Cholesterol/p. | ELISA ($OD_{405}$ nm) | Rating |
|---|---|---|---|---|---|
| 1 | N740 chest 1 | FP | 6.7 | 0.066 | – |
| 2 | N740 chest 2 | FP | 10.3 | 0.020 | – |
| 3 | Case 3 abdomen | FP | 5.7 | 0.084 | – |
| 4 | 6320 abdomen 1 | FS | 3.3 | 0.099 | – |
| 5 | 6320 abdomen 2 | FP | 20.3 | 0.036 | – |
| 6 | 6320 chest 1 | FS | 16.9 | 0.181 | – |
| 7 | S96 chest 2 | FP | 8.7 | 0.057 | – |
| 8 | S96 abdomen 3 | AP | 14.6 | 0.140 | – |
| 9 | N744 chest | FP | 1.2 | 0.561 | 2+ |
| 10 | N744 abdomen | AP | 4.4 | 0.391 | + |
| 11 | TO chest | FP | 2.8 | 0.526 | 2+ |
| 12 | To abdomen | FP | 11.3 | 0.236 | + |
| 13 | N745 | FS | 2.1 | 0.391 | + |
| 14 | N749-1 | CO | 3.0 | 0.340 | + |
| 15 | N749-2 | FP | 8.0 | 0.203 | + |
| 16 | S102-chest | FS | 1.7 | 0.738 | 3+ |
| 17 | S102-abdomen | FS | 1.4 | 0.554 | 2+ |
| 18 | 6345 | FP | 26.6 | 0.072 | – |
| 19 | N751 chest | FS | 6.8 | 0.806 | 4+ |
| 20 | N751 abdomen | FP | 8.8 | 0.348 | + |
| 21 | 6344 chest | FP | 17.2 | 0.242 | + |
| 22 | 6344 abdomen | FP | 23.5 | 0.157 | – |
| 23 | normal 5y chest | N | 1.0 | 0.132 | – |
| 24 | normal abdomen | N | 0.0 | 0.163 | – |

13

```
Lesions
    FP : fibrous plaque
    FS : fatty streaks
    AP : atheromatous plaque
    CO : calcification
    N  : normal
Criteria of rating
    ln terms of ELISA (OD405 nm) values:
            >0.8        : 4+
             0.6 - 0.8 : 3+
             0.4 - 0.6 : 2+
             0.2 - 0.4 : +
            <0.2        : -
Cholesterol/p.
    (cholesterol/protein) = (mg/dl)/(mg/ml)
```

## Claims

**Claims for the following Contracting States : AT, BE, CH, DE, FR, GB, IT, LI, LU, NL, SE**

1. A monoclonal antibody designated as 125H or 131B obtainable from hybridoma cell lines FERM BP 1675 and 1676 respectively.

2. A monoclonal antibody according to claim 1 wherein the monoclonal antibody is labelled with an enzyme or a radioisotope.

3. A monoclonal antibody according to claim 2 wherein the radioisotope is [125]I.

4. A process for preparing a monoclonal antibody designated as 125H or 131B which comprises the steps of immunizing a non-human mammal with a solution containing a human arteriosclerosis related antigen, fusing antibody producing lymhocytes from said animal with myeloma cells, screening for an anti-human arteriosclerosis antibody producing hybridoma FERM BP 1675 and FERM BP 1676, respectively, and cultivating the hybridoma cell line FERM BP 1675 and 1676, respectively, and recovering said antibody.

5. A process according to claim 4 wherein the solution containing a human arteriosclerosis related antigen is the serum of a familial hypercholesterolemic patient.

6. A process according to claim 4 wherein the solution containing a human arteriosclerosis related antigen is the serum of a patient suffering from cerebral infarction or myocardial infarction.

7. A process according to claim 4 wherein the solution containing a human arteriosclerosis related antigen is a homogenate of an arteriosclerotic lesion.

8. A process according to claim 7 wherein the arteriosclerotic lesion is as swelling portion of tunica intima.

9. A reagent for diagnosis of human arteriosclerosis comprising a monoclonal antibody as defined in claim 2.

10. A reagent for imaging diagnosis of human arteriosclerosis comprising a monoclonal antibody as claimed in claim 2 which is labelled with a radioisotope.

11. A diagnostic reagent according to claim 9 or 10 wherein the human arteriosclerosis is myocardial infarction or cerebral infarction.

12. A diagnostic reagent according to claim 11 wherein the radioisotope is $^{123}$I, $^{125}$I or $^{131}$I.

13. A diagnostic reagent according to claim 11 wherein the radioisotope is $^{111}$In.

14. A diagnostic reagent according to claim 11 wherein the radioisotope is $^{99m}$Tc.

**Claims for the following Contracting States : ES, GR**

1. A process for preparing a monoclonal antibody designated as 125H or 131B which comprises cultivating the hybridoma cell lines FERM BP 1675 and 1676, respectively, and recovering said antibody.

2. A process according to claim 1 wherein the monoclonal antibody is labelled with an enzyme or a radioisotope.

3. A process according to claim 2 wherein the monoclonal antibody is labelled with the radioisotope $^{125}$I.

4. A process for preparing a monoclonal antibody designated as 125H or 131B which comprises the steps of immunizing a non-human mammal with a solution containing a human arteriosclerosis related antigen, fusing antibody producing lymhocytes from said animal with myeloma cells, screening for an anti-human arteriosclerosis antibody producing hybridoma FERM BP 1675 and FERM BP 1676, respectively, and cultivating the hybridoma cell line FERM BP 1675 and 1676, respectively, and recovering said antibody.

5. A process according to claim 4 wherein the solution containing a human arteriosclerosis related antigen is the serum of a familial hypercholesterolemic patient.

6. A process according to claim 4 wherein the solution containing a human arteriosclerosis related antigen is the serum of a patient suffering from cerebral infarction or myocardial infarction.

7. A process according to claim 4 wherein the solution containing a human arteriosclerosis related antigen is a homogenate of an arteriosclerotic lesion.

8. A process according to claim 7 wherein the arteriosclerotic lesion is as swelling portion of tunica intima.

9. A method for preparing a reagent for diagnosis of human arteriosclerosis by preparing a reagent comprising a monoclonal antibody as defined in claim 2.

10. A method for preparing a reagent for imaging diagnosis of human arteriosclerosis by preparing a reagent comprising a monoclonal antibody as defined in claim 2 which is labelled with a radioisotope.

11. A method according to claim 9 or claim 10 wherein the human arteriosclerosis is myocardial infarction or cerebral infarction.

12. A method according to claim 11 wherein the radioisotope is $^{123}$I, $^{125}$I or $^{131}$I.

13. A method according to claim 11 wherein the radioisotope is $^{111}$In.

14. A method according to claim 11 wherein the radioisotope is $^{99m}$Tc.

**EP 0 334 076 B1**

**Patentansprüche**
**Patentansprüche für folgende Vertragsstaaten : AT, BE, CH, DE, FR, GB, IT, LI, LU, NL, SE**

1. Monoklonaler Antikörper, der als 125H oder 131B bezeichnet wird und aus Hybridzellen der Linien FERM BP 1675 bzw. 1676 erhältlich ist.

2. Monoklonaler Antikörper nach Anspruch 1, wobei der monoklonale Antikörper mit einem Enzym oder einem Radioisotop markiert ist.

3. Monoklonaler Antikörper nach Anspruch 2, wobei das Radioisotop $^{125}$I ist.

4. Verfahren zur Herstellung eines monoklonalen Antikörpers, der als 125H oder 131B bezeichnet wird, welches die Schritte der Immunisierung eines nicht-menschlichen Säugetiers mit einer Lösung, die ein Antigen, das mit menschlicherArteriosklerose im Zusammenhang steht, enthält; des Verschmelzens von Antikörper-produzierenden Lymphozyten von diesem Tier mit Myelomzellen; des Screening nach Hybridzelle FERM BP 1675 bzw. FERM BP 1676, das einen Antikörper gegen menschliche Arteriosklerose produziert; des Kutivierens der Hybridzelle der Linie FERM BP 1675 bzw. 1676; sowie des Isolierens des Antikörpers, umfaßt.

5. Verfahren nach Anspruch 4, bei dem die Lösung, die ein Antigen, das mit menschlicher Arteriosklerose im Zusammenhang steht, enthält, das Serum eines familiären hypercholesterinämischen Patienten ist.

6. Verfahren nach Anspruch 4, bei dem die Lösung, die ein Antigen, das mit menschlicher Arteriosklerose im Zusammenhang steht, enthält, das Serum eines Patienten ist, der an zerebralem Infarkt oder myokardialem Infarkt leidet.

7. Verfahren nach Anspruch 4, bei dem die Lösung, die ein Antigen, das mit menschlicher Arteriosklerose im Zusammenhang steht, enthält, ein Homogenat einer arteriosklerotischen Läsion ist.

8. Verfahren nach Anspruch 7, bei dem die arteriosklerotische Läsion ein schwellender Teil der Tunika intima ist.

9. Reagens zur Diagnose von menschlicher Arteriosklerose, das einen monoklonalen Antikörper, wie er in Anspruch 2 definiert ist, enthält.

10. Reagens zur bildgebenden Diagnose menschlicher Arteriosklerose, das einen monoklonalen Antikörper nach Anspruch 2 enthält, welcher mit einem Radioisotop markiert ist.

11. Diagnostisches Reagens nach Anspruch 9 oder 10, bei dem die menschliche Arteriosklerose ein myokardialer Infarkt oder ein zerebraler Infarkt ist.

12. Diagnostisches Reagens nach Anspruch 11, bei dem das Radioisotop $^{123}$I, $^{125}$I oder $^{131}$I ist.

13. Diagnostisches Reagens nach Anspruch 11, bei dem das Radioisotop $^{111}$In ist.

14. Diagnostisches Reagens nach Anspruch 11, bei dem das Radioisotop $^{99m}$Tc ist.

**Patentansprüche für folgende Vertragsstaaten : ES, GR**

1. Verfahren zur Herstellung eines monoklonalen Antikörper, der als 125H oder 131B bezeichnet wird, welches das Kultivieren von Hybridzellen-Linien FERM BP 1675 bzw. 1676 und Isolieren des Antikörpers umfaßt.

2. Verfahren nach Anspruch 1, bei dem der monoklonale Antikörper mit einem Enzym oder einem Radioisotop markiert ist.

3. Verfahren nach Anspruch 2, bei dem der monoklonale Antikörper mit dem Radioisotop $^{125}$I markiert ist.

EP 0 334 076 B1

**4.** Verfahren zur Herstellung eines monoklonalen Antikörpers, der als 125H oder 131B bezeichnet wird, welches die Schritte der Immunisierung eines nicht-menschlichen Säugetiers mit einer Lösung, die ein Antigen, das mit menschlicher Arteriosklerose im Zusammenhang steht, enthält; des Verschmelzens von Antikörper-produzierenden Lymphozyten von diesem Tier mit Myelomzellen; des Screening nach Hybridzelle FERM BP 1675 bzw. FERM BP 1676, die einen Antikörper gegen menschliche Arteriosklerose produziert; des Kutivierens der Hybridzelle der Linie FERM BP 1675 bzw. 1676; sowie des Isolierens des Antikörpers; umfaßt.

**5.** Verfahren nach Anspruch 4, bei dem die Lösung, die ein Antigen, das mit menschlicher Arteriosklerose im Zusammenhang steht, enthält, das Serum eines familiären hypercholesterinämischen Patienten ist.

**6.** Verfahren nach Anspruch 4, bei dem die Lösung, die ein Antigen, das mit menschlicher Arteriosklerose im Zusammenhang steht, enthält, das Serum eines Patienten ist, der an zerebralem Infarkt oder myokardialem Infarkt leidet.

**7.** Verfahren nach Anspruch 4, bei dem die Lösung, die ein Antigen, das mit menschlicher Arteriosklerose im Zusammenhang steht, enthält, ein Homogenat einer arteriosklerotischen Läsion ist.

**8.** Verfahren nach Anspruch 7, bei dem die arteriosklerotische Läsion ein schwellender Teil der Tunika intima ist.

**9.** Verfahren zur Herstellung eines Reagenzes für die Diagnose menschlicher Arteriosklerose, bei dem ein Reagens, das einen monoklonalen Antikörper, der in Anspruch 2 definiert ist, enthält, hergestellt wird.

**10.** Verfahren zur Herstellung eines Reagenzes für eine bildgebenden Diagnose menschlicher Arteriosklerose, bei dem ein Reagens, das einen monoklonalen Antikörper nach Anspruch 2 enthält, welcher mit einem Radioisotop markiert ist, hergestellt wird.

**11.** Verfahren nach Anspruch 9 oder 10, bei dem die menschliche Arteriosklerose ein myokardialer Infarkt oder ein zerebraler Infarkt ist.

**12.** Verfahren nach Anspruch 11, bei dem das Radioisotop $^{123}$I, $^{125}$I oder $^{131}$I ist.

**13.** Verfahren nach Anspruch 11, bei dem das Radioisotop $^{111}$In ist.

**14.** Verfahren nach Anspruch 11, bei dem das Radioisotop $^{99m}$Tc ist.

**Revendications**
**Revendications pour les Etats contractants suivants : AT, BE, CH, DE, FR, GB, IT, LI, LU, NL, SE**

**1.** Anticorps monoclonal désigné par 125H ou 131B pouvant être obtenu à partir de la lignée cellulaire d'hybridome FERM BP 1675 ou 1676, respectivement.

**2.** Anticorps monoclonal selon la revendication 1, l'anticorps monoclonal étant marqué par une enzyme ou un radio-isotope.

**3.** Anticorps monoclonal selon la revendication 2, dans lequel le radio-isotope est $^{125}$I.

**4.** Procédé de préparation d'un anticorps monoclonal désigné par 125H ou 131B, qui comprend les étapes suivantes : immunisation d'un mammifère non humain avec une solution contenant un antigène associé à l'artériosclérose humaine, fusion de lymphocytes producteurs d'anticorps provenant dudit animal avec des cellules de myélome, criblage pour sélectionner un hybridome producteur d'anticorps antiartériosclérose humaine, respectivement FERM BP 1675 ou FERM BP 1676, et culture de la lignée cellulaire d'hybridome, respectivement FERM BP 1675 ou 1676, et extraction dudit anticorps.

**5.** Procédé selon la revendication 4, dans lequel la solution contenant un antigène associé à l'artériosclérose humaine est le sérum d'un patient atteint d'hypercholestérolémie familiale.

17

**6.** Procédé selon la revendication 4, dans lequel la solution contenant un antigène associé à l'artériosclérose humaine est le sérum d'un patient atteint d'infarctus cérébral ou d'infarctus du myocarde.

**7.** Procédé selon la revendication 4, dans lequel la solution contenant un antigène associé à l'artériosclérose humaine est un homogénat d'une lésion artérioscléreuse.

**8.** Procédé selon la revendication 7, dans lequel la lésion artérioscléreuse est une partie enflée de tunique interne.

**9.** Réactif pour le diagnostic d'artériosclérose humaine, comprenant un anticorps monoclonal tel que défini dans la revendication 2.

**10.** Réactif pour le diagnostic par visualisation d'artériosclérose humaine, comprenant un anticorps monoclonal tel que revendiqué dans la revendication 2 qui est marqué par un radio-isotope.

**11.** Réactif pour diagnostic selon la revendication 9 ou 10, l'artériosclérose humaine étant un infarctus du myocarde ou un infarctus cérébral.

**12.** Réactif pour diagnostic selon la revendication 11, dans lequel le radio-isotope est $^{123}$I, $^{125}$I ou $^{131}$I.

**13.** Réactif pour diagnostic selon la revendication 11, dans lequel le radio-isotope est $^{111}$In.

**14.** Réactif pour diagnostic selon la revendication 11, dans lequel le radio-isotope est $^{99m}$Tc.

**Revendications pour les Etats contractants suivants : ES, GR**

**1.** Procédé de préparation d'un anticorps monoclonal désigné par 125H ou 131B, qui comprend la culture de la lignée cellulaire d'hybridome FERM BP 1675 ou 1676, respectivement, et l'extraction dudit anticorps.

**2.** Procédé selon la revendication 1, dans lequel l'anticorps monoclonal est marqué par une enzyme ou un radio-isotope.

**3.** Procédé selon la revendication 2, dans lequel l'anticorps monoclonal est marqué par le radio-isotope $^{125}$I.

**4.** Procédé de préparation d'un anticorps monoclonal désigné par 125H ou 131B, qui comprend les étapes suivantes : immunisation d'un mammifère non humain avec une solution contenant un antigène associé à l'artériosclérose humaine, fusion de lymphocytes producteurs d'anticorps provenant dudit animal avec des cellules de myélome, criblage pour sélectionner un hybridome producteur d'anticorps antiartériosclérose humaine, respectivement FERM BP 1675 ou FERM BP 1676, et culture de la lignée cellulaire d'hybridome, respectivement FERM BP 1675 ou 1676, et extraction dudit anticorps.

**5.** Procédé selon la revendication 4, dans lequel la solution contenant un antigène associé à l'artériosclérose humaine est le sérum d'un patient atteint d'hypercholestérolémie familiale.

**6.** Procédé selon la revendication 4, dans lequel la solution contenant un antigène associé à l'artériosclérose humaine est le sérum d'un patient atteint d'infarctus cérébral ou d'infarctus du myocarde.

**7.** Procédé selon la revendication 4, dans lequel la solution contenant un antigène associé à l'artériosclérose humaine est un homogénat d'une lésion artérioscléreuse.

**8.** Procédé selon la revendication 7, dans lequel la lésion artérioscléreuse est une partie enflée de tunique interne.

**9.** Méthode de préparation d'un réactif pour le diagnostic d'artériosclérose humaine, par préparation d'un réactif comprenant un anticorps monoclonal tel que défini dans la revendication 2.

**10.** Méthode de préparation d'un réactif pour le diagnostic par visualisation d'artériosclérose humaine, par préparation d'un réactif comprenant un anticorps monoclonal tel que défini dans la revendication 2 qui est marqué par un radio-isotope.

**11.** Méthode selon la revendication 9 ou la revendication 10, dans laquelle l'artériosclérose humaine est un infarctus du myocarde ou un infarctus cérébral.

**12.** Méthode selon la revendication 11, dans laquelle le radio-isotope est $^{123}$I, $^{125}$I ou $^{131}$I.

**13.** Méthode selon la revendication 11, dans laquelle le radio-isotope est $^{111}$In.

**14.** Méthode selon la revendication 11, dans laquelle le radio-isotope est $^{99m}$Tc.

*Fig. I*

Subclasses of monoclonal
antibody 131B

IgA          IgM

IgG3                    IgG1

IgG2b        IgG2a

# Fig. 2

ANTIGENIC ACTIVITY BY DISEASE OF
MONOCLONAL ANTIBODY 131B

# *Fig. 3*

Fig. 4

## Fig. 5

N=61, Y=$4.20 \times 10^{-4}$ X + 0.46, R=0.10

TOTAL CHOLESTEROL (mg/d$\ell$)

ANTIGENIC ACTIVITY (OD405nm)

# Fig. 6

ANTIGENIC ACTIVITY BY LESION OF
MONOCLONAL ANTIBODY 125H

125H MoAb

| SAMPLE | LESION | ELISA (OD 405 nm) |
|--------|--------|-------------------|
| 2 | NORMAL | |
| 6 | FATTY STREAK | |
| 13 | FIBROUS PLAQUE | |
| 2 | ATHEROMATOUS PLAQUE | |
| 1 | COMPLICATED LESION | |

## Fig. 7

## Fig. 8

Fig. 9

Fig. 10

# Fig. 11